# EUROPEAN PATENT APPLICATION

(11) **EP 1 795 227 A1**
(43) Date of publication of application: **13.06.2007**
(21) Application number: 06255967.9
(22) Date of filing: 22.11.2006
(51) Int. Cl.: A61N 1/375, A61M 5/142, A61N 1/372

(54) **Compliant biocompatible packaging scheme based on NiTi shape memory alloys for implantable biomedical microsystems**

(30) Priority: 06.12.2005 US 294892
(71) Applicant: Nitinol Development Corporation, Fremont, California 94539 (US)
(72) Inventor: Krulevitch, Peter, Pleasanton, CA 94566 (US)
(74) Representative: Belcher, Simon James

(57) **Abstract**

A compliant biocompatible packaging scheme, (i.e., coating) for implantable medical device, particularly implantable biomedical microsystems is provided. In accordance with the present invention, the compliant biocompatible coating includes an optional dielectric undercoating and an overcoat comprising a NiTi shape memory alloy.

## Description

The present invention relates to implantable medical devices, and in particular to a compliant biocompatible packaging scheme, (i.e., coating) for implantable medical devices including the biomedical microsystems thereof. In accordance with the present invention, the compliant biocompatible coating includes a NiTi shape memory alloy. The inventive compliant biocompatible coating is formed on at least one surface of at least one biomedical microsystem of an implantable medical device. The at least one biomedical microsystem can be located on a surface of the implantable medical device itself and/or it can be located some distance from the implantable medical device. The at least one biomedical microsystem includes electronics that are formed on, and/or within, a substrate. In some embodiments, the substrate is a biocompatible substrate, while in others the substrate is a non-biocompatible substrate. In accordance with the present invention, the NiTi shape memory alloys protect the substrate from contact with the body.

Implantable medical devices with active components such as, for example, cochlear implants, pacemakers, defibrillators, and urinary control devices, are becoming commonplace. Other implantable systems on the horizon include, for instance, neurostimulators for pain control, programmable drug delivery devices, glucose sensors, deep brain stimulators for treating Parkinson's disease and epilepsy, and retinal prostheses. Packaging of such implantable medical devices is critical for these systems, as the systems must be compact, biocompatible, hermetic (both to protect electronics and ensure that the body is not exposed to internal elements of the device), and long lived (100 years for devices such as cochlear implants that are implanted as early as one year of age and are expected to function for the life of the implantee).

Nearly all of the currently available implantable systems use a titanium (Ti) can or brazed ceramic packages, which meet present requirements, but are large, rigid, and expensive to manufacture. Implantable applications of the future require miniature packages that conform to the anatomy surrounding the implant and can withstand deformation during implantation. Miniaturization is critical to reduce trauma during implantation, enabling minimally invasive procedures. As an example, visual prostheses of the future will be implanted entirely within the eye, and will be inserted through an approximately 5 mm incision in the sclera. Finally, as the number of implants increases, manufacturing costs must be reduced.

In light of the above, implantable medical devices based on microelectromechanical systems (MEMS) technology have been developed. For example, silicon-based devices with integrated microelectrode arrays for neurostimulation have been designed and fabricated. Moreover, a flex-circuit approach that enables integration of custom and off-the-shelf electronics with microelectrode arrays using polyimide as a substrate have been developed.

Despite these developments, no one has successfully demonstrated long-term viability of implantable biomedical microsystems because a suitable packaging approach has not been found. Inorganic coatings such as, for example, glass and silicon nitride have shown promise, but these coatings are generally brittle and inflexible. Organic polymeric coatings such as, for example, polyamide and silicone, break down when chronically implanted. The traditional welded Ti can approach is one option that has been adopted for MEMS-based drug delivery, but this approach defeats the purpose of miniaturization all together due to the large size of the Ti can package.

In view of the drawbacks mentioned above with implantable biomedical microsystems, there is a need for developing a packaging scheme (i.e., coating) that has long-term viability, and is flexible, rugged, biocompatible, robust, deformable and implantable. Moreover, a packaging scheme is needed that maintains the miniature properties of implantable biomedical microsystems.

The present invention provides a compliant biocompatible packaging scheme (i.e., coating) that is suitable for use in medical devices, particularly implantable medical devices that include biomedical microsystems. Not only is the inventive packaging scheme complaint and biocompatible, but it also has long-term viability, and is flexible, rugged, robust, deformable and implantable. Moreover, the inventive packaging scheme maintains the miniature properties of implantable biomedical microsystems. The term "implantable biomedical microsystem" includes the electronic components of an implantable medical device which are fabricated using MEMS technology. The electronic components that are fabricated by MEMS technology can be located on a surface of the implantable medical device itself, they can be located some distance from the implantable medical device or both. The electronic components typically, but not necessarily, include microelectrode arrays which include metallic electrodes that are connected to metallic pads via metallic traces.

Since most implantable medical devices and hence their corresponding biomedical microsystems are intended for prolonged use and directly interface with body tissues, body fluids, electrolytes, proteins, enzymes, lipids, and other biological molecules, the coating materials for such medical devices and biomedical microsystems must meet stringent biological and physical requirements. These requirements, as a minimum, include the following criteria: (1) the coatings must be flexible and elastic, so they conform to the biological structure without inducing detrimental stress; (2) the coatings must be hemocompatible, and thereby reduce or avoid formation of thrombus or emboli; (3) the coatings must be chemically inert to body tissue and body fluids; (4) the coatings must be mechanically durable and not crack when formed on medical devices and the biomedical microsystem; (5) the coatings must be continuous, without pinholes or gaps that could allow the penetration of bodily fluids; and (6) the coatings must have very low permeability to moisture.

The present invention provides a compliant biocompatible packaging scheme, which satisfies the above requirements, that includes at least a coating of a NiTi shape memory alloy. In some embodiments of the present invention, the inventive packaging scheme may also include an undercoating that includes a dielectric material that is tolerant to high deposition temperatures that are used in forming the NiTi shape memory alloy. By "high deposition temperature" it is meant that the optional dielectric material can withstand temperatures on the order of about 450°C or greater. Examples of high temperature resistant dielectrics that can be employed in the present invention include, but are not limited to: oxides, polyamides, nitrides and diamond. Typically, the optional dielectric material that is used as the undercoat material is a polyamide.

The NiTi shape memory alloy of the present invention is characterized as having the formula Ni₁₀₀₋ₓTiₓ, wherein x is an integer from about 45 to about 53. In some embodiments, the NiTi shape memory alloy may further include Cu, Pd, Pt, and other like alloying metals. Mixtures of these alloying metals are also contemplated herein. In a preferred embodiment of the present invention, the NiTi shape memory alloy is a substantially equal mixture of Ni and Ti. Such a NiTi shape memory alloy having substantially equal mixture of Ni and Ti is referred to as Nitinol (Nitinol is an acronym for Nickel Titanium Naval Ordnance Laboratory). The NiTi alloy may be crystalline or amorphous, with crystalline forms being highly preferred. Nitinol has the advantages that it is highly ductile, repeatedly tolerating very large deformations (as high as 8% strain) without the formation of cracks or dislocations, and it is biocompatible. A thin film Nitinol is well known to have the same properties as bulk Nitinol material.

The present invention, which provides a compliant biocompatible coating for implantable biomedical microsystems, will now be described in greater detail by referring to the following discussion and drawings that accompany the present application. The drawings, which are included with the present application, are provided for illustrative purposes and, as such, they are not drawn to scale. It is further observed that the drawings of the present invention illustrate the embodiment in which the NiTi shape memory alloy is formed on a top surface of a substrate. Although such an embodiment is described and illustrated, the NiTi shape memory alloy can be formed on the bottom surface of a substrate as well as the sides of the substrate. Thus, and in some embodiments, the top, side and bottom surfaces of a substrate can be coated with the NiTi shape memory alloy.

As stated above, the present invention relates to a compliant biocompatible packaging scheme, i.e., coating, that is useful in protecting at least the biomedical microsystems of an implantable medical device. The term "biomedical microsystem" is used throughout this application to denote the electronic components of an implantable medical device, which are fabricated using conventional MEMS technology. Thus, the electronic components employed in the present invention have features that are typically about 10 µm (10 microns) or less.

The inventive coating described herein is impermeable to water and gases and thus it is suitable for hermetic packaging. The inventive coating adds very little bulk or rigidity to the implantable device, enabling the production of extremely low profile devices that conform to the tissues that they interface with. The small size also reduces time for both the surgical procedure and patient recovery, and allows for more placement options. Moreover, the inventive coating is an already acceptable material for long-term chronic implants for devices such as stents. In addition, the deposition of the inventive coating inherently lends itself to low cost production in large quantities.

Embodiments of the present invention will now be described by way of example with reference to the accompanying drawings, in which:
FIG. 1 is a pictorial representation (through a cross sectional view) showing an optional dielectric material that can be applied to at least one surface of at least one biomedical microsystem of an implantable medical device; and
FIG. 2 is a pictorial representation (through a cross sectional view) showing the structure of FIG. 1 after a NiTi shape memory alloy has been applied as a topcoat covering the optional dielectric material that is formed over the biomedical microsystem.

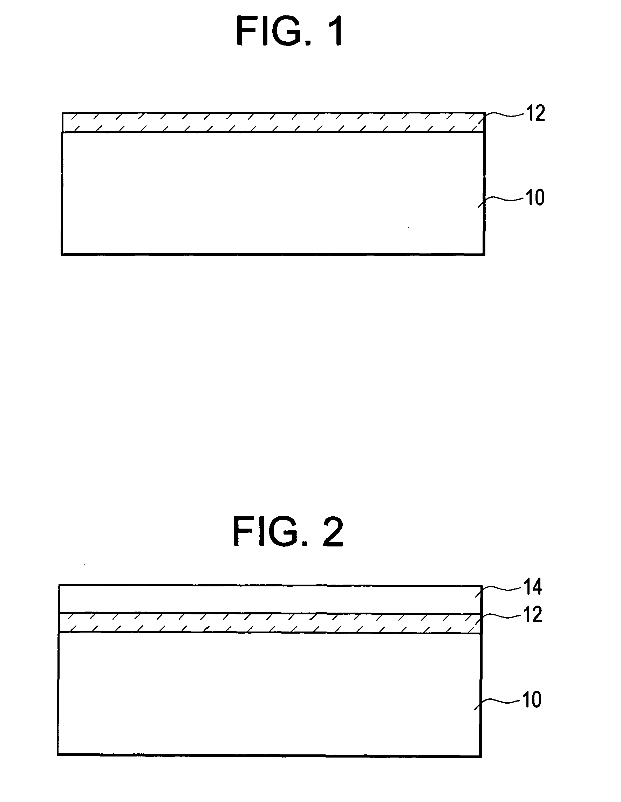

The present invention is now described in greater detail by referring to FIGS. 1-2, which show the processing steps of the present invention for forming the inventive coating on at least one biomedical microsystem of an implantable medical device. It is again observed that the drawings of the present invention illustrate the embodiment in which the NiTi shape memory alloy is formed on a top surface of a substrate. Although such an embodiment is described and illustrated, the NiTi shape memory alloy can be formed on the bottom surface of a substrate as well as the sides of the substrate. Thus, and in some embodiments, the top, side and bottom surfaces of a substrate can be coated with the NiTi shape memory alloy.

FIG. 1 illustrates the structure that is formed after providing a dielectric material 12 to a surface of a biomedical microsystem 10 of an implantable medical device. The biomedical microsystem 10 includes implantable electronic components (not shown) of the implantable medical device. The electronic components include, but are not limited to: circuitry (i.e., integrated circuits such as, for example, transistors, resistors, capacitors, etc.) and potentially surface mount electronics. The devices also include MEMS, which have electronics and mechanical components, such as membranes, cantilevers, resonators, sensors, and microelectrode arrays, comprising metallic electrodes that are contacted to metallic pads via metallic traces. The electronics are made using conventional MEMS technology which is well known to those skilled in the art.

The biomedical microsystem 10 may be located on, and/or encased within a substrate. The substrate may be non-biocompatible (such as glass, metallic and polymeric) or biocompatible, with biocompatible substrates being highly preferred. It is noted that non-biocompatible substrates can be used since the NiTi shape memory alloy protects materials that are not biocompatible from contact with the body. Of course it is beneficial if the substrates are biocompatible. The term "biocompatible" is used throughout this application to denote a material that is compatible with living tissue or a living organism by not being toxic or injurious and by not causing immunological reaction.

The biocompatible substrates are typically polymeric materials that are generally hydrophobic, flexible and which can conform to many different shapes, including curved surfaces. It is noted that the term "polymer" is used to denote a chemical compound with high molecular weight consisting of a number of structural units linked together by covalent bonds.

Illustrative examples of polymeric materials that can be used as the biocompatible substrate of the biomedical microsystem 10 include, but are not limited to: silicone polymers (i.e., organosiloxanes), polyurethanes, polyimides, parylene, fluoropolymers such as, for example, Teflon, polyolefins such as, for example, polyethylene or polypropylene, collagen, chitin, alginate, polyvinyl pyrrolidone, polyethylene glycol, polyethylene oxide, polyvinyl alcohol, polyglycol lactic acid, polylactic acid, polycaprolactone, polyamino acid, and a hydrogel such as, for example, carboxymethyl cellulose. Typically, polyimide polymers are used as the biocompatible substrate of the biomedical microsystems.

The biomedical microsystem 10 is a component of an implantable medical device, which is also biocompatible. By "implantable" it is meant that the medical device including the microsystem thereof can be inserted into a living site for medical usage. The term "biocompatible" has the same meaning as provided above. Suitable implantable medical devices that can be used in the present invention include, but are not limited to: cochlear implants, visual prostheses, neurostimulators, pacemakers, defibrillators, pain control devices, drug delivery devices, sensors for monitoring pressure in the brain, eye, and cardiovascular system, accelerometers for activity monitoring, RF ID tags, urinary control devices, muscular stimulators, and deep brain stimulators.

In addition to the biomedical microsystem 10, the structure shown in FIG. 1 also includes a dielectric material 12. The dielectric material 12 is optional and is not needed in all instances. When present, dielectric material 12 provides additional protection to the electronics and other components of an implantable medical device.

The optional dielectric material 12 comprises a high temperature tolerant material that can withstand the deposition temperature of the NiTi film that will be applied thereon. That is, the optional dielectric material 12 must be stable at temperature of about 450°C or greater. Illustrative examples of such high temperature tolerant dielectric materials include, but are not limited to: oxides, polyimides, polyamides, nitrides, diamond, and combinations and multilayers thereof. In some embodiments of the present invention, the optional dielectric material 12 comprises a polyamide.

When present, the optional dielectric material 12 is formed utilizing a conventional deposition process including, for example, chemical vapor deposition (CVD), plasma enhanced chemical vapor deposition (PECVD), spin-on coating, dip coating, spray coating, evaporation, atomic layer deposition (ALD) and chemical solution deposition. The thickness of the optional dielectric material 12 may vary depending upon the type of dielectric employed, the number of layers comprising the dielectric material 12, and the technique used in forming the same. Typically, the optional dielectric material 12 has an as deposited thickness from about 0.1 to about 10 µm (about 0.1 to about 10 microns), with an as deposited thickness from about 0.5 to about 2 µm (about 0.5 to about 2 microns) being even more typical.

Next, and as shown in FIG. 2, a NiTi shape memory alloy 14 is formed either atop the optional dielectric material 12, if present, or atop a surface of the biomedical microsystem 10. It is noted that the NiTi shape memory alloy 14 can be applied only to the biomedical microsystem or it can be applied to other components of the implantable medical device, including the implantable medical device itself. It is again worth mentioning that the NiTi shape memory alloy can also be applied such that it covers all surfaces of the device (top, bottom, and sides), or only a portion of the device that needs a flexible biocompatible coating.

The NiTi shape memory alloy 14 of the present invention is characterized as having the formula Ni₁₀₀₋ₓTiₓ, wherein x is an integer from about 45 to about 53. In some embodiments, the NiTi shape memory alloy 14 may further include Cu, Pd, Pt and other like alloying metals. Combinations of such alloying metals are also contemplated herein. In a preferred embodiment of the present invention, the NiTi shape memory alloy 14 is a substantially equal mixture ofNi and Ti, i.e., Nitinol (50 atomic % Ni and 50 atomic % Ti). The NiTi shape memory alloy 14 may be crystalline or amorphous, with crystalline forms being highly preferred.

The NiTi shape memory alloy 14 is formed utilizing deposition processes well known in the art. For example, the NiTi shape memory alloy 14 can be formed by sputtering, evaporation, metalorgano deposition, electroplating and other like deposition processes. Deposition is generally performed at a temperature of about 450°C or greater, with a deposition temperature from about 400° to about 600°C being even more general. Alternatively, deposition can be performed at low temperature (near room temperature) to form an amorphous film, with subsequent annealing near 500°C producing a crystalline film. Typically, the NiTi shape memory alloy 14 is formed by sputtering.

The thickness of the NiTi shape memory alloy 14 may vary depending upon the technique used in forming the same as well as the exact composition of the alloy itself. Typically, the NiTi shape memory alloy 14 has an as deposited thickness from about 0.1 to about 20 µm (about 0.1 to about 20 microns), with a thickness from about 1 to about 5 µm (about 1 to about 5 microns) being even more typical.

In some embodiments of the present invention, the NiTi shape memory alloy 14 may be annealed after deposition. When such an embodiment is employed, the annealing of the NiTi shaped memory alloy 14 is performed in an inert gas ambient such as He, Ar, N₂ or a mixture thereof at a temperature from about 450° to about 600°C, with an annealing temperature from about 475° to about 525° C being even more typical. The annealing is generally performed for a time period from about 10 to about 120 minutes, with an annealing time period from about 30 to about 60 minutes being even more typical. Annealing is used in applications in which a crystalline NiTi shape memory alloy is needed. In instances when an amorphous NiTi shape memory alloy 14 is needed, the annealing step may be omitted. Annealing can also be used to modify the properties of a crystalline film. Sometimes a quench (sudden temperature drop) is needed after annealing to lock in the desired properties.

It is noted that at least the NiTi shape memory alloy 14 and optionally the dielectric material 12 form a compliant biocompatible packaging scheme (i.e., coating) that is suitable for use in implantable medical devices that include biomedical microsystems. Not only is the inventive packaging scheme compliant and biocompatible, but it also has long-term viability, and is flexible, rugged, robust, deformable and implantable. Moreover, the inventive packaging scheme maintains the miniature properties of implantable biomedical microsystems.

It is further noted that the inventive packaging scheme described above also satisfies the following criteria: (1) it is flexible and elastic, so it conforms to the biological structure without inducing detrimental stress; (2) it is hemocompatible, and thereby reduces and avoids formation of thrombus or emboli; (3) it is chemically inert to body tissue and body fluids; (4) it is mechanically durable and does not crack when formed on medical devices and the biomedical microsystem; (5) the coatings must be continuous, without pinholes or gaps that could allow the penetration of bodily fluids; and (6) the coatings must have very low permeability to moisture.

## Claims

1. A coated article comprising:
a NiTi shape memory alloy located on at least one surface of at least one biomedical microsystem of an implantable medical device.

2. The coated article of Claim 1 wherein said at least one biomedical microsystem comprises metallic electrodes that are connected to metallic pads by way of metallic traces.

3. The coated article of Claim 1 wherein said at least one biomedical microsystem comprises electronics and sensors.

4. The coated article of Claim 1 wherein said at least one biomedical microsystem is located on said implantable medical device, located a distance from said implantable medical device or both.

5. The coated article of Claim 1 wherein said at least one biomedical microsystem is located on a biocompatible polymeric material, encased within a biocompatible polymeric material or both.

6. The coated article of Claim 1 further comprising a dielectric material located underneath said NiTi shape memory alloy covering said at least one biomedical microsystem, said dielectric material is tolerant to a deposition temperature of about 450°C or greater.

7. A coated article comprising
a dielectric material located on at least one surface of at least one biomedical microsystem of an implantable medical device, said dielectric material is tolerate to a deposition temperature of about 450°C or greater; and
a NiTi shape memory alloy covering said dielectric material.

8. The coated article of Claim 1 or 7, wherein said NiTi shape memory alloy has the formula Ni₁₀₀₋ₓTiₓ, wherein x is an integer from about 45 to about 53.

9. The coated article of Claim 1 or 7, wherein said NiTi shape memory alloy further comprises at least one of Cu, Pd, or Pt.

10. The coated article of Claim 1 or 7, wherein said NiTi shape memory alloy comprises a substantially equal mixture ofNi and Ti.

11. The coated article of Claim 1 or 7, wherein said NiTi shape memory alloy is crystalline.

12. The coated article of Claim 1 or 7, wherein said NiTi shape memory alloy is amorphous.

13. The coated article of Claim 1 or 7, wherein said dielectric material is an oxide, a polyamide, a nitride, or diamond.

14. A coated article comprising
an implantable medical device that includes at least one biomedical microsystem;
a polyamide on a least one surface of said at least one biomedical microsystem; and
a shape memory alloy comprising a substantially equal mixture of Ni and Ti located on said polyamide.
